# EUROPEAN PATENT APPLICATION

(11) **EP 1 153 544 A2**
(43) Date of publication of application: **14.11.2001**
(21) Application number: 01304141.3
(22) Date of filing: 08.05.2001
(51) Int. Cl.: A01N 33/12, A01N 25/34

(54) **Antimicrobial cleansing composition and wipe**

(30) Priority: 11.05.2000 US 203412 P
(71) Applicant: UNILEVER PLC, London EC4P 4BQ (GB); UNILEVER N.V., 3013 AL Rotterdam (NL)
(72) Inventor: Lunsmann, Walter Joseph, c/o Unilever Home &, Trumbull, Connecticut 06611 (US); Villa, Virogilio Barba, c/o Unilever Home &, Trumbull, Connecticut 06611 (US)
(74) Representative: Elliott, Peter William

(57) **Abstract**

A mild, aqueous antimicrobial cleansing composition is disclosed which contains little or no volatile alcohol and does not leave a sticky residue during drying. The composition may be used alone or in combination with shampoos, lotions, body cleansers, and the like. In another embodiment, impregnating a water insoluble substrate with the inventive composition provides a disposable wipe. The process of making and using the inventive wipe is also described.

## Description

This invention relates generally to an aqueous antimicrobial cleansing composition, and more particularly to an insoluble substrate saturated with the composition.

When travelling, traditional sink cleansing of the face and hands is not possible or convenient, and therefore people have relied on hand sanitizers or cleansing wipes (e.g. Wet Ones®) for antimicrobial protection. For these products to be effective as antimicrobial cleansers, the formulations have contained alcohol, resulting in products which are very harsh to the skin. Alcohol based antimicrobial products have been reported to present an increased health risk due to the damage to the skin's integrity with usage over time. (Soap/Cosmetics/Chemical Specialties, November, 1998)

The prior art teaches e.g in USP 5,888,524 issued to D. Cole on March 30, 1999 and USP 5,700,842 issued to D. Cole on Dec. 23, 1997; both of which are incorporated herein by reference, that an advantageous method which avoids the use of alcohol to create an effective antimicrobial wipe requires the use of antimicrobial actives e.g. from the family of halogenated aromatic hydrocarbons such as triclosan or triclocarban. These compounds antimicrobial properties are usually effective in the presence of anionic and amphoteric surfactants typically found in cleansing compositions and, therefore, can be effectively used for antimicrobial efficacy in traditional cleansing wipe formulations. The art also teaches that the use of amides in combination with e.g. triclosan is required to physically stabilise the active in the formulation. The disadvantage of this formulation approach is the very sticky feel of the product in use (during evaporation or "dry-down") due to the triclosan and amide precipitating onto the skin during the evaporation stage. This very sticky after-feel is usually unacceptable to the consumer.

The prior art has also shown the effectiveness of another class of antimicrobial compounds, i.e. water soluble or dispersible cationic antimicrobial actives such as quaternary ammonium compounds e.g. benzethonium or benzalkonium salts. In the past, benzethonium chloride has been used as an effective antimicrobial active in wound treatment (FDA monograph 21 CFR 333 and 369), dental applications and opthalmic applications. This art, however, does not disclose or suggest the use of benzethonium chloride in personal cleansing applications, or in combination with typical surfactant systems.

Such antimicrobial use is, in fact, severely compromised by the presence of anionic and amphoteric surfactants; compounds which have been traditionally required for effective cleansing in wipe applications. Prior art wipes containing cationic antimicrobial actives, such as quaternary ammonium compounds require alcohol for effectiveness and acceptable skin feel during dry-down. For example, products such as Wet Ones' ® antibacterial wipes, Wet Ones' ® Lunchkin's ®, and Bath & Body Works' ® antibacterial cleansing wipes have used benzethonium chloride as an antibacterial co-active but have also had to rely on the use of alcohols in the formulation to achieve both effective, antibacterial efficacy and acceptable skin feel in-use (during the "dry-down" period).

The present invention describes an antimicrobial cleansing composition, a water insoluble substrate or wipe, which is impregnated with the inventive composition; and the process of making and using the inventive wipe. The inventive composition demonstrates antimicrobial efficacy from a water soluble or dispersible bactericidal agent, preferably a cationic antimicrobial compound, more preferably a quaternary ammonium compound, or a salt or precursor thereof, most preferably from a compound selected from benzethonium methyl, benzethonium and benzalkonium water-soluble salts. The inventive composition and inventive wipe also achieves effective, mild cleansing from one or more nonionic surfactants, and achieves an acceptable skin feel in-use, free from stickiness, without any volatile alcohol (bp < 210°) present in the formulation. Moreover, the inventive composition contains less than 1 % of any of the compounds selected from an amphoteric surfactant, an anionic surfactant, a halogenated aromatic hydrocarbon, an amide, and a volatile alcohol with a boiling point of under 210°C. Preferably the inventive composition contains less than 0.5% of these aforementioned compounds. Most preferably the composition contains none of these compounds.

This invention relates to an antimicrobial cleansing composition, a water insoluble substrate or wipe, which is impregnated with the inventive composition; and the process of making and using the inventive wipe.

One aspect of the present invention is an aqueous antimicrobial, cleansing composition comprising an effective amount of at least one water soluble or dispersible antimicrobial agent, or a precursor or salt thereof; at least one nonionic surfactant; and wherein said composition contains less than 1 % of an compound selected from an amphoteric surfactant, an anionic surfactant, a halogenated aromatic hydrocarbon, an amide, and a volatile alcohol with a boiling point of under 210 C. Preferably the inventive composition does not feel sticky upon dry-down and contains less than 0.5% of any of these ingredients. Most preferably the inventive compositions contains none of these ingredients.

Preferably the batericidal agent is a cationic antimicrobial compound, more preferably a quaternary ammonium compound, or a salt or precursor thereof. Suitable quaternary ammonium compounds include benzethonium chloride, methyl benzethonium chloride, benzalkonium chloride and the like. Most preferable is a compound selected from benzethonium and benzalkonium water-soluble salts. Suitable salts can include, but are not limited to chlorides, iodides, bromides and phosphates and the like. In the case of benzethonium and benzalkonium chlorides, an effective concentration range would be 0.01 to 5, more preferably 0.05 to 0.5 weight percent.

The other aspects of the present invention will be described for use as antimicrobial compositions for incorporating into conventional wet wipes. However, it is to be understood, that the antimicrobial compositions may be used in the manufacture of other consumer products such as, for example, shampoos, soaps, cleansing agents, detergents, lotions, and the like.

### Antimicrobial Agents

It has been discovered that hydrophilic antimicrobial agents may be used in aqueous compositions to provide homogeneous antimicrobial compositions if the compositions are prepared according to the present invention. The antimicrobial composition of the different aspects of the present invention includes an effective amount of a water soluble or dispersible (hydrophilic) antimicrobial agent, combined with an aqueous mixture which includes a nonionic surfactant.

A wide range of hydrophilic antimicrobial agents which provide antimicrobial compositions may be used in the different aspects of the present invention. The antimicrobial composition may include a single hydrophilic antimicrobial agent or a combination of two or more hydrophilic antimicrobial agents. Preferably, the hydrophilic antimicrobial agent of the present invention is a broad spectrum antimicrobial agent. For example, suitable hydrophilic antimicrobial agents include benzethonium chloride, methylbenzethonium chloride, benzalkonium chloride, chlorohexidine gluconate, p-chloro-m-xylenol,hexachlorophene, and the like, and combinations, salts, and precursors thereof.

The hydrophilic antimicrobial agent may be present in the composition in any amount which provides an antimicrobial composition. However, if the amount of the hydrophilic antimicrobial agent is too high, the composition may be cloudy and irritating to the skin of the wearer. Moreover, if the amount of the hydrophilic antimicrobial agent is too low, the composition may not be antimicrobial. As set forth above, the antimicrobial effectiveness of the antimicrobial composition can be determined by testing the composition against several known microorganisms. It has been found that antimicrobial compositions of the present invention which include from about 0.01 to about 5 weight percent, preferably from about 0.05 to about 0.5 weight percent and more preferably from about 0.1 to about 0.4 weight percent of the hydrophilic antimicrobial agent based on the total weight of the composition are effective against most microorganisms while not irritating the skin. It has also been found that the antimicrobial composition of the different aspects of the present invention is particularly effective when it contains from about 0.01 to about 5 weight percent and more preferably from about 0.05 to about 0.5 weight percent benzethonium chloride based on the total weight of the composition.

The inventive antimicrobial compositions may also include additional elements such as, for example, emollients, perfuming agents, chelating agents, cleansing agents, foam stabilizers, preservatives, protectants, and the like, to enhance the performance of the compositions.

Accordingly, the different aspects of the present invention provide antimicrobial compositions which include hydrophilic antimicrobial agents in an aqueous environment. In a particular aspect, the composition of the present invention demonstrate evidence of antimicrobial activity using the zone of inhibition method based on a traditional Bauer-Kirby Method used for antibiotic sensitivity assays for clinical microbiology procedures as described in e.g. the National Committee for Clinical Laboratory standards. Susceptible organisms were shown to include the following: E. coli (ATCC #10536), S. epidermis (ATCC #12228), and S. aureus (ATCC #6538). Other pontially susceptible organisms are disclosed in the art. See e.g. (1) Cosmetic and Drug Preservation - Principles and Practice, Edited by Jon J. Kabara, Marcel Dekker, Inc. New York, 1984, pp734-735, and (2) Product Literature for Hyamine 1622 (Benzethonium chloride), Lonza Specialty Chemicals; which are herein incorporated by reference. Such aqueous, antimicrobial compositions are particularly useful in premoistened wipes and cosmetic products such as liquid soaps, shampoos, and lotions, and the like.

### Method of Manufacturing the Wipe

The wet wipes are saturated or otherwise impregnated with the antimicrobial compositions of the present invention, as described herein, by any suitable means such as coating, spraying, dipping, soaking or the like as are well known to those skilled in the art. The amount of the antimicrobial composition which may be added to the wet wipes may vary depending upon the type of material being used to provide the wet wipe, the type of container being used to store the wet wipes, and the desired end use of the wet wipe. Generally, each wet wipe can contain from about 50 to about 600 weight percent and preferably from about 100 to about 450 weight percent of the antimicrobial composition based on the dry weight of the wipe. In a particular aspect, wherein the wet wipe is made from a hydroentangled material comprising polymeric microfibers, the amount of the antimicrobial composition contained within the wet wipe is from about 50 to about 400 weight percent and preferably from about 100 to 250 weight percent based on the dry weight of the wet wipe. If the amount of liquid is less than the above-identified range, the wet wipe may be too dry and may not adequately perform. If the amount of liquid is greater than the above-identified range, the wet wipe may be oversaturated and soggy and the liquid may pool in the bottom of the container.

Accordingly, the different aspects of the present invention can also advantageously provide an antimicrobial, cleansing wet wipe which, when compared to conventional wet wipes, has improved antimicrobial effectiveness and is nonirritating to the user. In particular, the different aspects of the present invention can provide an antimicrobial cleansing wet wipe, which is wetted with an aqueous antimicrobial composition which includes a hydrophilic antimicrobial agent. Such wet wipes can advantageously be used for baby wipes, hand wipes, face wipes, cosmetic wipes, household wipes, industrial wipes and the like.

In yet another aspect, the present invention concerns an antimicrobial cleanser or lotion which includes the antimicrobial compositions described herein. Generally, the cleanser or lotion contains from about 1.0 to about 95 weight percent and preferably from about 5.0 to about 25 weight percent of the antimicrobial effective composition based on the total weight of the cleanser or lotion. The cleansers and lotions of the present invention may also include additional elements such as for example, emollients, oils, emulsifiers, silicones, fatty alcohols, fatty acids, perfuming agents, chelating agents, cleansing agents, foam stabilizers, preservatives, protectants, sunscreen and anti-acne compounds and the like, to enhance the performance of the cleansers and lotions. When compared to conventional cleansers and lotions, the antimicrobial lotion of the present invention has improved antimicrobial effectiveness and is nonirritating to the user. Such cleansers or lotions can advantageously be used for body washes, baby lotions, hand lotions, face lotions and the like.

### Nonionic Surfactants

The nonionic surfactant of the present invention comprises 0.01 to 10% by weight, preferably 0.1 to 1% by wt. of the antimicrobial cleansing composition. The nonionic which may be used includes in particular the reaction products of compounds having a hydrophobic group and a reactive hydrogen atom, for example aliphatic alcohols, acids, amides or alkyl phenols with alkylene oxides, especially ethylene oxide either alone or with propylene oxide. Specific nonionic detergent compounds are alkyl (C₆-C₂₂) phenols-ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxides, and the like.

The nonionic may also be a sugar amide, such as a polysaccharide amide. Specifically, the surfactant may be one of the lactobionamides described in U.S. Patent No. 5,389,279 titled "Compositions comprising nonionic glycolipid surfactants" issued on Feb. 14, 1995 to Au et al. which is hereby incorporated by reference or it may be one of the sugar amides described in Patent No. 5,009,814 titled "Use of n-polyhydroxyalkyl fatty acid amides as thickening agents for liquid aqueous surfactant systems" issued on Apr. 23, 1991 to Kelkenberg, hereby incorporated into the subject application by reference.

Other surfactants which may be used are described in U.S. Patent No. 3,723,325 to Parran Jr. and alkyl polysaccharide nonionic surfactants as disclosed in U.S. Patent No. 4,565,647 titled "Foaming surfactant compositions", issued on Jan. 21, 1986 to Llenado, both of which are also incorporated into the subject application by reference.

Preferred alkyl polysaccharides are alkylpolyglycosides of the formula

R²O(CₙH₂ₙO)ₜ(glycosyl)ₓ

wherein R² is selected from alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

Preferably the nonionic surfactant is selected from a condensation product of a saccharide polymer and a C8 to C12 alcohol. The nonionic more preferably is selected from decyl glucoside, lauryl glucoside, coco-glucoside and combinations thereof.

The inventive composition does not feel sticky on dry-down. Stickiness may be qualitatively assessed by a panel of persons who apply the composition to their skin, or may be quantitatively assessed by art recognized test methods. Suitable quantitative methods include measuring the force required to remove a probe from the surface wetted by the composition during or after evaporation, or by weighing an insoluble powder residue adhering to a surface wetted by the composition during or after evaporation, or the like.

The inventive composition and inventive wipe also achieves effective, mild cleansing from one or more nonionic surfactants, and achieves an acceptable skin feel in-use, free from stickiness, without any volatile alcohol bp < 210° present in the formulation. Moreover, the inventive composition contains less than 1 % of any compound selected from the group consisting of an amphoteric surfactant, an anionic surfactant, a halogenated aromatic hydrocarbon, an amide, and a volatile alcohol with a boiling point of under 210°C. Preferably the inventive composition contains less than 0.5% and most preferably it does not contain any of these compounds.

### Water Insoluble Substrate:

The inventive antimicrobial cleansing wipe contains a water insoluble substrate as a component. By "water insoluble" is meant the substrate does not dissolve or readily break apart upon immersion in water. A wide variety of materials can be used as the substrate. The following non-limiting characteristics are desirable: (i) sufficient wet strength for use, (ii) sufficient abrasivity, (iii) sufficient loft and porosity, (iv) sufficient thickness, and (v) appropriate size.

Non-limiting examples of suitable insoluble substrates which meet the above criteria include non-woven substrates, woven substrates, hydro-entangled substrates, air entangled substrates and the like. Preferred embodiments employ non-woven substrates since they are economical and readily available in a variety of materials. By non-woven is meant that the layer is comprised of fibers which are not woven into a fabric but rather are formed into a sheet, particularly a tissue. The fibers can either be random (i.e., randomly aligned) or they can be carded (i.e. combed to be oriented in primarily one direction). Furthermore, the non-woven substrate can be composed of a combination of layers of random and carded fibers.

Non-woven substrates may be comprised of a variety of materials both natural and synthetic. By natural is meant that the materials are derived from plants, animals, insects or byproducts. By synthetic is meant that the materials are obtained primarily from various man-made materials or from material that is usually a fibrous web comprising any of the common synthetic or natural textile-length fibers, or mixtures thereof.

Non-limiting examples of natural materials useful as components in the present invention are silk fibers, keratin fibers and cellulosic fibers. Non-limiting examples of keratin fibers include those selected from wool fibers, camel hair fibers, and the like. Non-limiting examples of cellulosic fibers include those selected from wood pulp fibers, cotton fibers, hemp fibers, jute fibers, flax fibers, and mixtures thereof. Wood pulp fibers are preferred while all cotton fibers (e.g. cotton pads) are normally avoided.

Non-limiting examples of synthetic materials useful as components in the present invention include those selected from acetate fibers, acrylic fibers, cellulose ester fibers, modacrylic fibers, polyamide fibers, polyester fibers, polyolefin fibers, polyvinyl alcohol fibers, rayon fibers and mixtures thereof. Examples of some of these synthetic materials include acrylics such as Acrilan®, Creslan®, and the acrylonitrile-based fiber, Orlon®; cellulose ester fibers such as cellulose acetate, Arnel®, and Acele®; polyamides such as Nylons (e.g., Nylon 6, Nylon 66, Nylon 610 and the like; polyesters such as Fortrel®, Kodel®, and the polyethylene terephthalate fibers, Dacron®; polyolefins such as polypropylene, polyethylene; polyvinyl acetate fibers and mixtures thereof.

Non-woven substrates made from natural materials consist of webs or sheets most commonly formed on a fine wire screen from a liquid suspension of the fibers. Substrates made from natural materials useful in the present invention can be obtained from a wide variety of commercial sources.

Non-woven substrates made from synthetic material useful in the present invention can also be obtained from a wide variety of commercial sources, such as e.g. PGI 7027 65/35 rayon polyester from PGI Company (Greenville, NC) and Buckeye 6009 92 % wetlaid wood pulp with 8% latex binder from Buckeye Company (Nashville, TN) and the like.

Most preferred as a component substrate for purposes of this invention are non-woven substrates, especially blends of rayon/polyester and ratios of 10:90 to 90:10, preferably ratios of 20:80 to 80:20, optimally 40:60 to 70:30 by weight. A most useful substrate is a 65:35 rayon/polyester non-woven wipe article.

The antimicrobial wet wipes may appear in several different forms. For example the wet wipes may be in the form of a stack of moistened sheets which have been packaged in a plastic container. The wet wipes may also be in a folded or unfolded configuration. In addition, the wet wipes may be in the form of continuous webs of material which include perforations to separate the individual wet wipes from the continuous web. Such continuous webs may be wound into rolls and also packaged in plastic containers. Such wet wipes can be used for baby wipes, hand wipes, household cleaning wipes, industrial wipes and the like.

Anywhere from 1 to 100, preferably from 5 to 50 single wipes may be stored within a dispensing pouch or container, preferably a moisture impermeable pouch or container. During storage and between dispensing, the pouch or container is preferably resealable. Single wipe containing pouches may also be employed.

### METHOD OF USING THE TREATED WIPE ARTICLES

For treatment of the user's skin or hair, the treated wipe is saturated with water, and is applied to a surface (e.g., a skin surface) via topical application to release or deposit an effective amount of the aqueous liquid composition to perform the desired antimicrobial cleansing function. The amount of water-insoluble functional ingredient delivered from the wipe and frequency of topical application can vary widely, depending upon the individual user's needs. With respect to personal application to the skin, such application can range from about once per day to about four times daily, preferably from about twice per day to about three times daily. The number of wipes used per application can range from I to about 4 wipes, preferably I to about 2 wipes. The amount of water-insoluble functional ingredient deposited on each wipe is generally from about 1.0 mg to about 100 mg per wipe. The treated wipes of the present invention can also be used prophylactically by administrating to healthy skin surfaces to guard from or prevent undesired skin conditions and/or infections using the dosing regimen described above.

Except in the operating and comparative examples, or where otherwise explicitly indicated, all numbers in this description indicating amounts of material ought to be understood as modified by the word "about".

The following, non-limiting examples will more fully illustrate the embodiments of this invention. All parts, percentages and proportions referred to herein and in the appended claims are by weight unless otherwise illustrated.

### Examples 1-8

Comparative and inventive wipes were made with the compositions and nonwoven substrates described below. The wipes were then tested for antimicrobial efficacy and by panellists for skin feel stickiness acceptability versus an alcohol based control (Example 8) such as Wet One's® antimicrobial moist wipes, manufactured by Playtex Inc.

Formulation of the compositions were accomplished as follows: initially the oil based ingredients are emulsified with the emulsifier e.g. polysorbate 20 in a separate submix - then e.g. polysorbate, DMDM Hydantoin, fragrance and tocopheryl acetate are added to the submix vessel and blended. Next, in the main mix vessel, all the salts are dissolved in room temperature (20 - 25 C) water, e.g. sodium citrate, citric acid, Versene and benzethonium chloride, and blended until uniform. Next the emollients (e.g. glycerine) are added to the main mix vessel, followed by the surfactant(s) (e.g. decyl glucoside) to the main mix vessel and blended until uniform. Then the oil-based submix is added to the main vessel and blended until uniform. The measured pH was in the range of 5 to 5.5.

The above solutions were next homogeneously dosed onto the nonwoven substrate using the following conditions: 6.6 grams per 5.5" x 7.5" sheet, Buckeye 6009 92% wetlaid wood pulp with 8% latex binder, 100 grams/sq. meter; or 4.2 grams per 5.5" x 7.5" sheet of PGI 7027 nonwoven substrate, 65/35 rayon/ polyester.

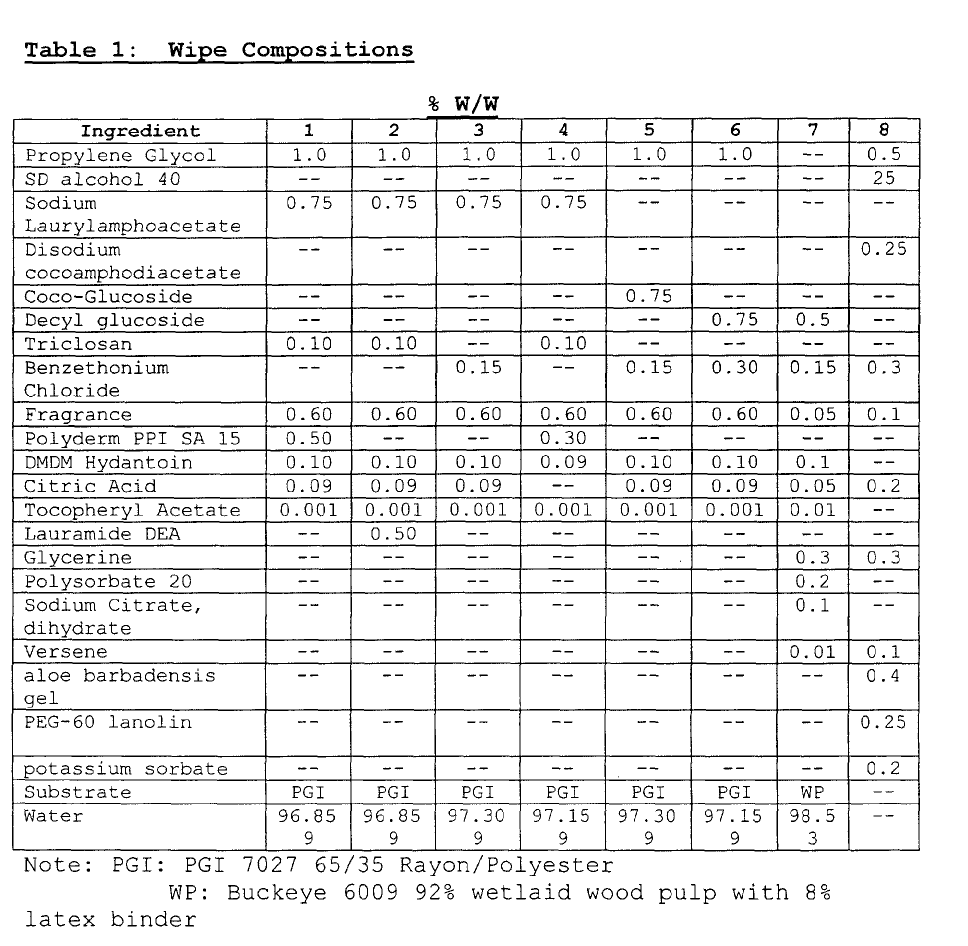

**Table 2:**

| **Wipe Performance** | | | |
|---|---|---|---|
| Example | Kind | Antimicrobial Efficiency | Skin Feel Stickiness |
| 1 | Comparative | Positive | Fail |
| 2 | Comparative | Positive | Fail |
| 3 | Comparative | None | Pass |
| 4 | Comparative | None | Pass |
| 5 | Inventive | Positive | Pass |
| 6 | Inventive | Positive | Pass |
| 7 | Inventive | Positive | Pass |
| 8 | Control | -- | -- |

## Claims

1. An aqueous, mild, antimicrobial cleansing composition, comprising:
a. an effective amount of a water soluble or dispersible antimicrobial agent, or a precursor or salt thereof;
b. a nonionic surfactant; and
wherein said composition contains less than 1 % of any compound selected from an amphoteric surfactant, an anionic surfactant, a halogenated aromatic hydrocarbon, an amide, and a volatile alcohol with a boiling point of under 210°C.

2. The composition of claim 1 wherein said antimicrobial agent is a cationic compound, or a salt or precursor thereof.

3. The composition of claim 2 wherein said cationic compound is a quaternary ammonium compound having at least one aromatic group.

4. The composition of claim 3 wherein said aromatic quaternary ammonium compound is selected from benzethonium chloride, benzalkonium chloride and methyl benzethonium chloride.

5. The composition of claim 3 wherein said aromatic quaternary ammonium compound is present in a concentration range of 0.05 to 0.5 weight percent.

6. The composition of claim 1 wherein the nonionic surfactant is selected from a condensation product of a saccharide polymer and a C8 to C12 alcohol.

7. The composition of claim 6 wherein the nonionic surfactant is selected from decyl glucoside, lauryl glucoside, coco-glucoside and mixtures thereof.

8. The composition of claim 1 wherein the composition does not contain any compound selected from an amphoteric surfactant, an anionic surfactant, a halogenated aromatic hydrocarbon, an amide, and a volatile alcohol with a boiling point of under 210°C.

9. The composition of claim 1 wherein said composition contains a benefit agent.

10. The composition of claim 9 wherein said benefit agent is selected from a polyhydric alcohol, a polyol, vitamins, skin emollients and anti acne compounds.

11. A mild antimicrobial, cleansing wipe, comprising:
a. a water insoluble substrate;
b. an aqueous, mild, antimicrobial cleansing composition, said composition including an effective amount of a water soluble or dispersible bactericidal agent, or a precursor or salt thereof; and a nonionic surfactant; and
wherein said composition contains less than 1 % of any compound selected from an amphoteric surfactant, an anionic surfactant, a halogenated aromatic hydrocarbon, an amide, and a volatile alcohol with a boiling point of under 210°C.

12. The wipe of claim 11 wherein the water insoluble substrate is selected from wet-laid, hydroentangled, meltblown and air-laid nonwovens.

13. The wipe of claim 11 wherein said bactericidal agent is a quaternary ammonium compound, or a salt or precursor thereof.

14. The wipe of claim 13 wherein said quaternary ammonium compound has at least one aromatic group.

15. The wipe of claim 14 wherein said aromatic quaternary ammonium compound is selected from benzethonium chloride, benzalkonium chloride and methyl benzethonium chloride.

16. The wipe of claim 14 wherein said aromatic quaternary ammonium compound is present in a concentration range of 0.05 to 0.5 weight percent.

17. The wipe of claim 11 wherein the nonionic surfactant is selected from a condensation product of a saccharide polymer and a C8 to C12 alcohol.

18. The wipe of claim 17 wherein the nonionic surfactant is selected from decyl glucoside, lauryl glucoside, coco-glucoside and mixtures thereof.

19. The wipe of claim 11 wherein the composition does not contain any compound selected from an amphoteric surfactant, an anionic surfactant, a halogenated aromatic hydrocarbon, an amide, and a volatile alcohol with a boiling point of under 210°C.

20. The wipe of claim 11 wherein said composition contains a benefit agent.

21. The wipe of claim 11 wherein said benefit agent is selected from a polyhydric alcohol, a polyol, vitamins, skin emollients sunscreen compounds and anti-acne compounds.

22. The process of making a mild antimicrobial, cleansing wipe, comprising the step of coating a water insoluble substrate with an aqueous, mild, antimicrobial cleansing composition, said composition including an effective amount of a water soluble or dispersible bactericidal agent, or a precursor or salt thereof; and
a nonionic surfactant; and
wherein said composition contains less than 1% of any compound selected from an amphoteric surfactant, an anionic surfactant, a halogenated aromatic hydrocarbon, an amide, and a volatile alcohol with a boiling point of under 210°C.

23. The process of making a mild antimicrobial, cleansing wipe, comprising the step of coating a water insoluble substrate with an aqueous, mild, antimicrobial cleansing composition, said composition including an effective amount of a water soluble or dispersible bactericidal agent, or a precursor or salt thereof; and a nonionic surfactant; and wherein said composition contains less than 1% of a compound selected from an amphoteric surfactant, an anionic surfactant, a halogenated aromatic hydrocarbon, an amide, and a volatile alcohol with a boiling point of under 210°C.

24. The process of using a mild antimicrobial, cleansing wipe, comprising the step of applying the wipe to the skin to remove dirt and oil, said composition including an effective amount of a water soluble or dispersible bactericidal agent, or a precursor or salt thereof; and a nonionic surfactant; and wherein said composition contains less than 1% of a compound selected from an amphoteric surfactant, an anionic surfactant, a halogenated aromatic hydrocarbon, an amide, and a volatile alcohol with a boiling point of under 210°C.
